# EUROPÄISCHE PATENTSCHRIFT

(11) **EP 0 493 677 B1**
(45) Veröffentlichungstag und Bekanntmachung des Hinweises auf die Patenterteilung: **18.03.1998**
(21) Anmeldenummer: 91120184.6
(22) Anmeldetag: 26.11.1991
(51) Int. Cl.: A61K 31/025, A61K 31/535

(54) **Verwendung einer Perfluorocarbon(PFC)-Mixtur zur Herstellung einer Behandlungsflüssigkeit zum Wiederanlegen (Entfalten) abgelöster Netzhaut an die Aderhaut des Auges**
Use of a solution for the preparation of a medicament for the reattachment of a detached retina to the choroid
Utilisation d'une solution pour la préparation d'un médicament pour le recollage d'une rétine décollée à la choroide

(30) Priorität: 03.01.1991 DE 4100059
(43) Veröffentlichungstag der Anmeldung: 08.07.1992
(73) Patentinhaber: Chiron Adatomed Pharmazeutische und Medizintechnische Gesellschaft mbH, 85609 Dornach (DE)
(72) Erfinder: Meinert, Hasso, Prof. Dr., W-7900 Ulm (DE)
(74) Vertreter: Nöth, Heinz, Dipl.-Phys.

(56) Entgegenhaltungen:
- EP-A- 0 089 232
- EP-A- 0 231 091
- EP-A- 0 415 263
- WO-A-89/00848
- DE-A- 2 555 408
- US-A- 4 366 169
- CHEMICAL ABSTRACTS, Band 110, Nr. 15, 10. April 1989, Seite 46, Zusammenfassung Nr. 128319t, Columbus, Ohio, US; S.L. VOROB'EV et al.: "Effects of oncotic agents as ingredients in perfluorocarbon emulsions on the isolated heart", & KHIM.-FARM. ZH. 1989, 23(1), 53-6
- OPHTHALMIC SURGERY, Band 20, Nr. 4, April 1989; M. NABIH et al.: "Experimental evaluation of perfluorophenanthrene as a high specific gravity vitreous substitute: a preliminary report"
- INTERNATIONAL COURSE ON INTERNAL TAMPONADE IN VITREORETINAL SURGERY, ABSTRACTS: note book. RAVENNA, Italy, 31/10-1/11.1992
- CLUB JULES GONIN, XVIII Meeting, Vortrag Nr. 97 (Seite 73/74), VIENNA, Austria VELIKAY, M. et al. "Perfluorodecalin and perfluorooctylbromide highly purified versus industrial products. An experimental study for short and extended time tolerance"
- JOURNAL OF VITREORETINA Vol. 1, June 1992, Seiten 17-19 FALCO, L., et al. "Comparison of different perfluorocarbon liquids"
- BULL. SOC. BELGE OPHIALMOL.,Band 238,1990, Seiten 145-150; C.CLAES et. al.:"The use of perfluorocarbon liquids in vitreous surgery"

## Beschreibung

Die Erfindung betrifft die Verwendung einer Perfluorcarbon(PFC)-Mixtur zur Herstellung einer Behandlungsflüssigkeit zum Wiederanlegen (Entfalten) abgelöster Netzhaut an die Aderhaut (Choroidea) des Auges, enthaltend wenigstens ein flüssiges Perfluorocarbon (PFC).

Eine derartige Behandlungsflüssigkeit ist aus der US-PS 4 490 351 bekannt.

Der Einsatz von flüssigen Perfluorocarbonen bei der Behandlung der Netzhautablösung und von großen Rissen in der Netzhaut hat sich aufgrund der hohen chemischen Stabilität und der Dichten im Bereich von 1,5 - 1,8 g/cm³ dieser Stoffe empfohlen. Bei der Netzhautentfaltung werden nach Entfernen des Glaskörpers die flüssigen PFCs in das Auge eingebracht, und während der Patient auf dem Rücken liegt, drücken diese Flüssigkeiten aufgrund ihrer Dichte die Netzhaut bzw. die mit Rissen behaftete Netzhaut wieder an das Aderhautgewebe des Auges. Nach einer Verweilzeit von mehreren Stunden wird das PFC wieder abgesaugt und durch ein anderes Medium, beispielsweise Methylsilikonöl ersetzt, da sich die PFCs nicht langzeitverträglich verhalten. Dies liegt an der hohen Dichte und auch an der Toxizität der PFCs gegenüber den Augenbestandteilen. In diesem Zusammenhang wird noch auf folgende Literatur hingewiesen:

H. Laqua, K. Lucke, M.H. Foerster "Entwicklung und gegenwärtiger Stand der Silikonölchirurgie" in Klin. Mbl. Augenheilk. 192, 1988, S. 277 - 283; A. Kampik "Prophylaxe und Behandlung der proliferativen Vitreoretinopathien" in Z. prakt. Augenheilkd. 7 S. 323 - 326 (1986); Stanley Chang, Emin Ozmert, Neal J. Zimmermann "Intraoperative Perfluorcarbon liquids in the Management of Proliferative Vitreoretinopathy in "American Journal of Ophthalmology 106 (Dec. 1988) S. 668 - 674; Stanley Chang, "Low Viscosity Liquid Fluorochemicals in Vitreous Surgery" in American Journal of Ophthalmology 103 (January 1987) S. 38 - 43; Anselm Kampik "Klinik und Pathogenese der Windenblütenablatic" in Z. prakt. Augenheilkd. 4 (1983) S. 371 - 378; Klaus Lucke "Vitreoretinale Chirurgie bei komplizierten Netzhautablösungen" in Z. prakt. Augenheilkd. 9 (1988), S. 137 - 147.

Aus BULL. SOC. BELGE Optalmol., Band 238, 1990, Seiten 145 - 150; C. Claes et al.: "The use of perfluorocarbon liquids in vitreous surgery" ist es bekannt, Perfluorocarbone zu verwenden, bei denen alle Wasserstoffatome durch Fluor substituiert sind. Ferner wurden PFC-Gase in der Netzhautchirurgie für die Netzhauttamponade verwendet. Ferner ist in der EP-A-0 089 232 darauf hingewiesen, daß neben der vollständigen Substitution der Wasserstoffatome die nicht vollständige Beseitigung aller Wasserstoffatome zugelassen werden kann, wenn die wesentlichen Eigenschaften der flüssigen Perfluorcarbone nicht beeinträchtigt werden, insbesondere wenn aktive Wasserstoffatome die Toxizität der Verbindungen nicht kritisch anheben. In der EP-A-0 415 263 sind als Heteroatome Stickstoff und Sauerstoff enthaltene perfluorierte heterozyklische Perfluorcarbone zur Verwendung in Blutersatzmitteln beschrieben. Schließlich ist aus Ophtalmic Surgery, Band 20, Nr. 4, April 1989, M. Nabih et al.: "Experimental evaluation of perfluorophenanthrene as a high specific gravity vitreous substitute: a preliminary report" bekannt, flüssiges Perfluorophenanthren als möglichen Glaskörperersatz in der Glaskörperchirurgie und bei der Netzhautentfaltung einzusetzen.

Aufgabe der Erfindung ist es, für die eingangs dargestellte Verwendung eine Behandlungsflüssigkeit zu schaffen, die langzeitverträglich ist, d.h. nichttoxisch ist gegenüber den bei der Entfaltungsbehandlung beteiligten Augenkomponenten.

Diese Aufgabe wird durch die im Patentanspruch 1 angegebenen Merkmale gelöst.

Hochreine PFCs lassen sich bevorzugt dadurch herstellen, daß das Ausgangs-PFC mit einem nukleophilen Agens, insbesondere einem sekundären Amin R₂NH und einer starken Base, insbesondere KOH oder NaOH über mehrere Tage am Rückfluß gehalten wird. Hierbei reagieren die vollständig fluorierten Verbindungen nicht. H-haltige und ungesättigte PFCs werden in einer Folge von Additions- und Eliminationsreaktionen umgewandelt. In Abhängigkeit von der jeweiligen Position der Doppelbindung entsteht eine Mischung aus stickstoffhaltigen Verbindungen. In diesem Drei-Phasensystem, bestehend aus PFC, Aminen und Wasser, gehen die durch Reaktion mit sekundärem Amin gebildeten Produkte in die Aminphase und können dabei von den unveränderten PFCs getrennt werden. Hochtoxische N-F-Verbindungen zersetzen sich gemäß den folgenden Gleichungen durch Reduktion mittels Jodidionen.

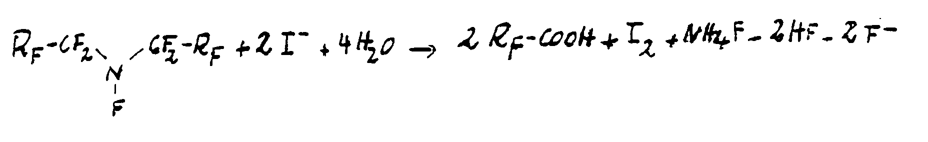

Auf diese Weise lassen sich PFCs, die in bekannter Weise in einem ECF- oder CoF₃-Prozeß synthetisiert worden sind, reinigen. Beispielsweise bei der Synthese von Perfluorodecalin durch CoF₃ -Fluorierung von Decalin oder Naphthalin bilden sich H- und/oder doppelbindungenhaltige Substanzen wie C₁₀F₁₈₋ₙHₙ, C₁₀F₁₆, C₁₀F₁₆₋ₙHₙ und C_{g}F₁₆₋ₙHₙ als Nebenprodukte.

Durch die oben angegebene Reinigung lassen sich diese Nebenprodukte auf weniger als 30 ppm verringern, so daß dann keine Toxizität mehr feststellbar ist.

In bevorzugter Weise enthält die PFC-Mixtur wenigstens ein PFC aus der Gruppe Perfluorodecalin, Perfluorocyclohexylmorpholin, Perfluoroctan und Perfluoroctylbromid in einem Anteil von 96 - 99,9 % und einen Anteil an PFCs mit niedriger Grenzflächenspannung, wofür Perfluoro-dimorpholinopropan oder Perfluoro-dimorpholinobutan oder eine perfluorierte, tricyclische Verbindung, wie Perfluoro-perhydrophenanthren in bevorzugter Weise in Frage kommen. Das PFC mit besonders niedriger Grenzflächenspannung ist bevorzugt in einem Anteil von 1 - 4 % in der Mixtur enthalten. Ferner erreicht man hierdurch eine Dampfdruckerniedrigung, so daß beim Glaskörperersatz im Auge verbleibendes PFC bei 37° C keinen zu hohen Dampfdruck aufweist und damit zu stark auf die Chorroidea wirkt. Ferner erreicht man durch eine derartige Mixtur eine Veränderung des Brechungsindex, so daß die Grenzfläche PFC/Gewebeflüssigkeit bei Durchführung der Operation deutlicher sichtbar ist.

PFCs besitzen eine sehr hohe Löslichkeit für Gase, die rein physikalischer Natur ist und somit dem Henry'schen Gesetz folgt. Die Löslichkeit der Gase ist daher bei konstanter Temperatur dem jeweiligen Partialdruck des Gases proportional. Die Löslichkeit hängt auch vom Molekulargewicht des Gases ab. Je höher das Molekulargewicht ist, um so besser ist seine Löslichkeit in PFC. Die bei Normalbedingungen flüssigen PFCs unterscheiden sich hinsichtlich ihres Lösevermögens dahingehend, daß mit fallendem Molekulargewicht ihr Lösevermögen zunimmt. Hierbei besitzen kettenförmige PFCs eine noch etwas höhere Löslichkeit als ringförmige PFCs.

Unter Normalbedingungen lösen flüssige PFCs 40 bis 55 Volumenprozent Sauerstoff und 100 bis 150 Volumenprozent CO₂.

In vorteilhafter Weise können daher gelöste Gase in der PFC-Mixtur zum Einsatz kommen, wobei diese Gase zum einen im Hinblick auf Gewebeversorgung bei der Netzhautentfaltung mit einem entsprechenden Gas, insbesondere gelöstem Sauerstoff, angereichert sind und zum anderen im Hinblick auf eine verbesserte Netzhautentfaltung durch Erzeugung eines temporär höheren Andruckes am darunterliegenden Gewebe mit einem gelösten, insbesondere chemisch inerten Gas, angereichert sind. Hierfür eignen sich bevorzugt gasförmige PFCs, wie vorzugsweise CF₄ oder C₂F₆ oder C₃F₈ oder C₄F₈ sowie auch Schwefelhexafluorid (SF₆).

Bei Normalbedingungen sind die gasförmigen PFCs sowie SF₆ sehr gut in flüssigen PFCs löslich. Ebenso wie die gasförmigen PFCs wird insbesondere SF₆ wegen seines hohen Molekulargewichts, seiner chemischen Inertheit, seiner kleinen Dielektrizitätskonstanten und seiner hohen Durchschlagsfestigkeit als gasförmiger Isolator in Hochspannungsgeneratoren und anderen elektrischen Geräten und wegen seiner geringen Wärmeleitfähigkeit als Temperaturisolator verwendet.

Die angesprochenen gasförmigen PFCs und das SF₆ bewirken bei der Netzhautentfaltung einen höheren Andruck der abgelösten Netzhaut an das darunterliegende Chorroidgewebe. Gegebenenfalls kann dieser erhöhte Andruck auch innerhalb eines nur relativ kurzen Zeitraumes zur Anwendung gebracht werden. Es reichen jeweils 0,5 bis 5,0 Volumenprozent, maximal 10 Volumenprozent, an zudotierten gasförmigen PFCs bzw. SF₆. Der erhöhte Andruck wird nicht nur durch das relativ hohe spezifische Gewicht der zudotierten Gase, sondern auch durch deren höheren Dampfdruck in der flüssigen PFC-Mixtur erreicht. In Abhängigkeit von der Menge der zudotierten gasförmigen PFCs bzw. des SF₆ und durch deren Partialdampfdrücke läßt sich der insbesondere im ersten Teil der Operationsphase erwünschte höhere Dampfdruck des Operationsmediums in Form der flüssigen PFC-Mixtur beliebig in seiner Höhe über einen gewünschten bestimmten Zeitraum hin variieren.

Um eine Netzhautentfaltung über einen längeren Zeitraum zu ermöglichen und gegebenenfalls eine nachträgliche Tamponade mit Silikonöl zu vermeiden, wird in bevorzugter Weise eine PFC-Emulsion in Wasser, die optisch homogen und durchsichtig ist, verwendet. In bevorzugter Weise kommt hierbei ein geeignetes Surfactant zur Anwendung, um die PFC-Komponente zu emulgieren. Bevorzugt kommt ein nichtionogener Emulgator zur Anwendung. In bevorzugter Weise beträgt die freie Emulgator(Surfactant)-Konzentration 1 bis 3 %. Als Surfactant eignet sich bevorzugt, insbesondere dann, wenn Perfluoroctylbromid als PFC zur Anwendung kommt, ein nicht ionogenes, biokompatibles Tensid, wie z. B. Polyalkylenglycol auf der Basis von Blockpolymeren aus Ethylen- und Propylenoxid, das unter dem Warenzeichen "Pluronic" bekannt ist.

Je nachdem, ob die PFC-Emulsion in Wasser direkt zur Netzhautentfaltung oder zur nachträglichen Substituierung des Kammerwassers verwendet werden soll, kann die Dichte der Emulsion dadurch eingestellt werden, daß der Anteil an PFC zwischen 10 bis 60 Gewichtsprozent beträgt. Die PFC-Komponente innerhalb dieses Konzentrationsanteils ist variabel. Als PFC-Komponente kommen neben Perfluoroctylbromid noch Perfluoroctan, Perfluorodecalin und Perfluorocyclohexylmorpholin innerhalb des angegebenen Konzentrationsanteils (10 bis 60 Gewichtsprozent) der Emulsion in Wasser mit einen Anteil von 96 bis 99,9 Vol.-% und einem weiteren Anteil vor 1 bis 4 Vol.-% Perfluoro-dimorpholinoalkan, insbesondere Perfluoro-dimorpholinopropan oder Perfluorodimorpho linobutan, oder einer perfluorierten, tricyclischen Verbindung, wie Perfluoro-perhydrophenanthren, in Frage, wobei die Vol.-%-Anteile auf das Volumen der PFC-Mixtur bezogen sind.

In vorteilhafter Weise kann hierbei die hohe Löslichkeit von Gasen, insbesondere von Sauerstoff, bei diesen PFCs ausgenützt werden. Man erreicht hiermit eine ausreichende Versorgung des Gewebes mit Sauerstoff. In bevorzugter Weise können den Emulsionen auch Adjuvantien, Metabolite, wie Ascorbinsäure, Retinol, und/oder Medikamente zugesetzt sein.

Der gewünschte osmotische Druck (Osmolarität ca. 350 nmol/kg) kann bevorzugt über isotonische Lösungen, insbesondere physiologische Salzkomponenten, eingestellt werden. Der onkotische Druck (ca. 4,42 kPa) wird in bevorzugter Weise durch Zugabe von Hydroxyethylstärke oder Dextran und/oder Hyaluronsäure eingestellt. Der gewünschte Dampfdruck (bei 37^{o} C ca. 5,06 kPa) kann z. B. durch die schon erwähnten zudotierten gasförmigen PFCs bei der Emulsion erreicht werden.

Die Emulsion enthält mithin ein entsprechendes Puffersystem, um den pH-Wert konstant zu halten.

Das spezifische Gewicht der Emulsion ist bevorzugt größer als 1 und kleiner als 1,6 eingestellt. Dies läßt sich durch den Gehalt an den jeweiligen PFCs erreichen. In der folgenden Tabelle sind Ausführungsbeispiele für verschiedene PFC-Mixturen, welche als Entfaltungsflüssigkeit bei der Wiederanlage abgelöster Netzhaut an die an der Haut des Auges angewendet werden können, aufgelistet sind.

| PFC-Mixturen | | | | |
|---|---|---|---|---|
| PFCs | Anteile (Volumen-%) | | | |
| Perfluorodecalin | 99 % | 98 % | 97 % | 96 % |
| oder | | | | |
| Perfluorocyclohexylmorpholin | dto | dto | dto | dto |
| oder | | | | |
| Perfluoroctan | dto | dto | dto | dto |
| oder | | | | |
| Perfluoroctylbromid | dto | dto | dto | dto |
| Perfluoro-dimorpholinopropan | 1 % (Vol) | 2 % (Vol) | 3 % (Vol) | 4 % (Vol) |
| oder | | | | |
| Perfluoro-dimorpholinobutan | dto | dto | dto | dto |
| oder | | | | |
| Perfluoro-perhydrophenanthren | dto | dto | dto | dto |

Die Mixturen können mit Hilfe herkömmlicher Mischer hergestellt werden.

Zur Herstellung der PFC-Emulsion in Wasser wird das PFC mit Hilfe eines geeigneten Surfactans, insbesondere eines nichtionogenen, biokompatiblen Surfactans (Emulgator) in einem Gaulin-Homogenisator mit Wasser homogenisiert und die Emulsion danach bei einer Porenweite bei ca. 400 Nanometer steril filtriert.

Im folgenden sind verschiedene Beispiele für optisch homogene, durchsichtige PFC-Emulsionen in Wasser angegeben. Für die in diesen Ausführungsbeispielen angegebene PFC-Komponente kann eine der in obiger Tabelle angegebenen PFC-Mixturen zum Einsatz kommen.

In der folgenden Tabelle sind verschiedene Ausführungsbeispiele für PFC-Emulsionen angegeben. Die Anteilsangaben sind % w/v.

| | | | | |
|---|---|---|---|---|
| PFC-Komponente | F-Cyclohexylmorpholin | 20,0 | F-Octylbromid | 50,0 |
| | F-Dimorpholinopropan | 2,0 | | |
| Tensid | Pluronic F-68 | 3,0 | | 6,0 |
| Onkotische Reaganzien | Hydroxyethylstärke | 3,0 | | 3,0 |
| Mineralsalze | NaCl | 0,58 | | 0,58 |
| | KCl | 0,034 | | 0,034 |
| | CaCl₂ | 0,020 | | 0,020 |
| | NaHCO₃ | 0,024 | | 0,024 |
| | MgCl₂ | 0,02 | | 0,02 |
| | Glukose | 0,17 | | 0,17 |
| | Ascorbinsäure | 0,020 | | 0,020 |
| | Harnstoff | 0,04 | | 0,04 |
| Wasser | | ad 100 ml | | ad 100 ml |

In den folgenden Tabellen 1 bis 4 und Bildern 1 bis 3 sind Dampfdruckkurven und Brechungsindizes von F-Dimorpholinoalkan/Perfluordekalin- und F-Dimorpholinopropan/Perfluoroktan-Lösungen angegeben. Die Ergebnisse belegen, daß das Verhalten der obengenannten Lösungen in allen Fällen nicht kollegativ (Additivität der Dampfdrücke bzw. der Brechungsindizes) ist und es sich folglich nicht, wie bisher für Perfluorcarbone allgemein angenommen, um ideale Lösungen handelt. Hierbei bedeuten n₁ den Molenbruch, P₂₅ den Dampfdruck bei 25^{o} C, P₃₇ den Dampfdruck bei 37^{o} C und n_{D}²⁵ den Brechungsindex bei 25^{o} C.

**Tab.1**

| **Dampfdrucke von F-Dimorpholinopropan/Perfluordekalin** | | |
|---|---|---|
| n₁ | p₂₅(Torr) | p₃₇(Torr) |
| - | 10,22 | 13,6 |
| 0,0038 | 10,05 | 13,27 |
| 0,0086 | 10,10 | 11,96 |
| 0,0180 | 9,16 | 11,82 |
| 0,0364 | 9,14 | 11,82 |
| 0,0382 | 8,53 | 11,70 |
| 1 | 5,72 | 6,30 |

**Tab.2**

| **Dampfdrucke von F-Dimorpholinopropan/Perfluoroktan** | | |
|---|---|---|
| n₁ | p₂₅(Torr) | p₃₇(Torr) |
| - | 28,36 | 49,40 |
| 0,0031 | 24,84 | 42,95 |
| 0,0092 | 27,66 | 46,53 |
| 0,0148 | 26,31 | 44,80 |
| 0,0420 | 27,24 | 45,83 |
| 1 | 5,72 | 6,30* |

| | | |
|---|---|---|
| * aus den Messungen für Perfluordekalin | | |

**Tab.3**

| **Brechungsindizes von F-Dimorpholinoalkan/Perfluordekalin** | | | | | |
|---|---|---|---|---|---|
| -propan- | | -butan- | | -pentan- | |
| n₁ | n_{D}²⁵ | n₁ | n_{D}²⁵ | n₁ | n_{D}²⁵ |
| - | 1,312₅ | - | 1,312₅ | - | 1,312₅ |
| 0,0106 | 1,312₀ | 0,0055 | 1,312₁ | 0,0225 | 1,312₂ |
| 0,0240 | 1,311₅ | 0,0137 | 1,311₈ | 0,0427 | 1,311₉ |
| 0,0369 | 1,311₈ | 0,0248 | 1,311₈ | 0,0864 | 1.311₆ |
| 0,0662 | 1,311₅ | 0,0439 | 1,311₈ | - | - |
| 0,1048 | 1,311₃ | - | - | - | - |

**Tab.4**

| **Brechungsindizes von F-Dimorpholinopropan/Perfluoroktan** | |
|---|---|
| n₁ | n_{D}²⁵ |
| - | 1,272 |
| 0,0057 | 1.272 |
| 0,0179 | 1,273 |
| 0,0930 | 1,278 |

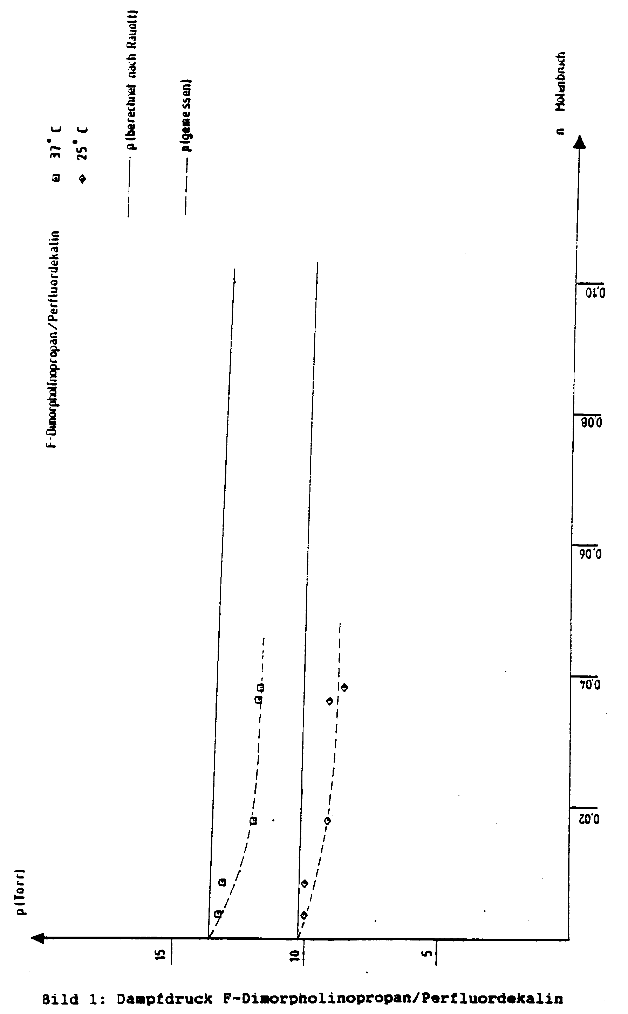

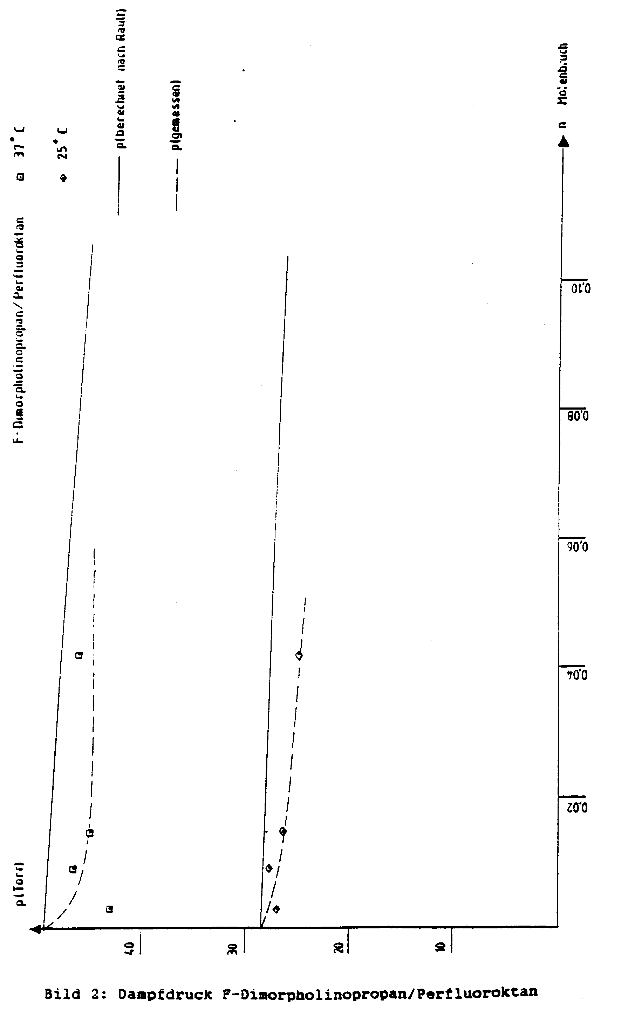

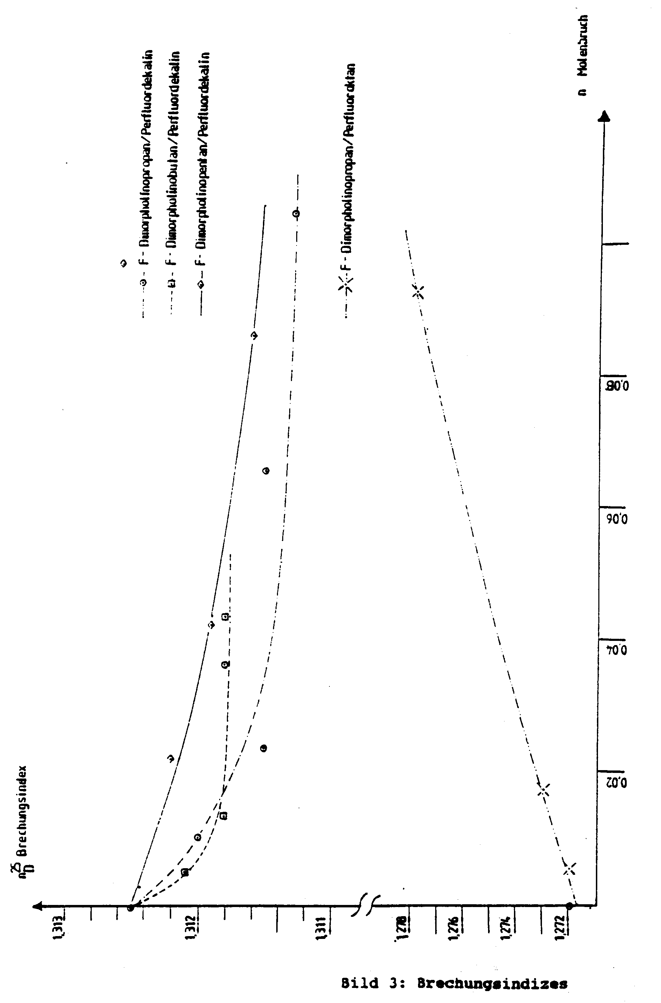

## Patentansprüche

1. Verwendung einer Perfluorcarbon(PFC)-Mixtur, enthaltend wenigstens ein flüssiges Perfluorcarbon, ausgenommen Perfluorophenanthren, die durch einen Reinigungsprozeß in eine Verbindung umgewandelt ist, die frei von C=C-Doppelverbindungen und C-H-Bindungen ist, zur Herstellung einer Behandlungsflüssigkeit zum Wiederanlegen (Entfalten) abgelöster Netzhaut an die Aderhaut eines Auges.

2. Verwendung nach Anspruch 1, dadurch gekennzeichnet, daß die PFC-Mixtur wenigstens ein PFC aus der Gruppe Perfluorodecalin, Perfluorocyclohexylmorpholin, Perfluoroctan, Perfluoroctylbromid aufweist.

3. Verwendung nach Anspruch 2, dadurch gekennzeichnet, daß das PFC aus der Gruppe Perfluorodecalin, Perfluorocyclohexylmorpholin, Perfluoroctan, Perfluoroctylbromid in einem Anteil von 96 bis 99,9 Vol.-% enthalten ist.

4. Verwendung nach einem der Ansprüche 1 bis 3, dadurch gekennzeichnet, daß die PFC-Mixtur einen Anteil von 1 bis 4 Vol.-% eines Perfluoro-dimorpholinoalkans oder eine perfluorierte, tricyclische Verbindung aufweist.

5. Verwendung nach Anspruch 4, dadurch gekennzeichnet, daß die perfluorierte, tricyclische Verbindung Perfluoro-perhydrophenanthren ist.

6. Verwendung nach Anspruch 4, dadurch gekennzeichnet, daß das Alkan Perfluoro-dimorphoniobutan oder Perfluoro-dimorpholinopropan ist.

7. Verwendung nach einem der Ansprüche 1 bis 6, dadurch gekennzeichnet, daß in der PFC-Mixtur ein Gas gelöst ist.

8. Verwendung nach Anspruch 7, dadurch gekennzeichnet, daß das gelöste Gas zur Gewebeversorgung dient.

9. Verwendung nach Anspruch 8, dadurch gekennzeichnet, daß als Gas Sauerstoff in der PFC-Mixtur gelöst ist.

10. Verwendung nach Anspruch 7, dadurch gekennzeichnet, daß das gelöste Gas zur Einstellung des Andruckes bei der Netzhautentfaltung dient.

11. Verwendung nach Anspruch 10, dadurch gekennzeichnet, daß ein oder mehrere gasförmige PFCs oder SF₆ in der PFC-Mixtur gelöst sind.

12. Verwendung nach einem der Ansprüche 1 bis 11, gekennzeichnet als PFC-Emulsion in Wasser.

13. Verwendung nach Anspruch 12, dadurch gekennzeichnet, daß die Emulsion hinsichtlich ihres osmotischen und onkotischen Druckes auf Augenglaskörperbedingungen eingestellt ist.

14. Verwendung nach Anspruch 12 oder 13, dadurch gekennzeichnet, daß die Emulsion hinsichtlich ihres osmotischen und onkotischen Druckes auf Kammerwasserbedingungen eingestellt ist.

15. Verwendung nach einem der Ansprüche 10 bis 14, dadurch gekennzeichnet, daß der Anteil an PFC-Mixtur in der Emulsion 10 - 60 Gewichtsprozent beträgt.

16. Verwendung nach einem der Ansprüche 10 bis 15, dadurch gekennzeichnet, daß der Emulgator ein nicht-ionogener Emulgator ist.

17. Verwendung nach einem der Ansprüche 10 bis 16, gekennzeichnet durch eine freie Emulgatorkonzentration von 1 bis 3 %.

18. Verwendung nach einem der Ansprüche 10 bis 17, dadurch gekennzeichnet, daß der Emulgator ein nicht-ionogenes biokompatibles Tensid ist.

19. Verwendung nach Anspruch 18, dadurch gekennzeichnet, daß das Tensid ein Ethylenoxid-Propylenoxid-Blockpolymer oder ein davon abgeleitetes Derivat ist.

20. Verwendung nach einem der Ansprüche 10 bis 19, dadurch gekennzeichnet, daß die Einstellung des osmotischen Druckes durch physiologische Salzkomponenten erfolgt.

21. Verwendung nach einem der Ansprüche 10 bis 20, dadurch gekennzeichnet, daß der onkotische Druck durch Zugabe wenigstens eines Stoffes der Gruppe Hydroxyethylstärke, Dextran, Hyaluronsäure eingestellt ist.

22. Verwendung nach einem der Ansprüche 10 bis 21, dadurch gekennzeichnet, daß das spezifische Gewicht der Emulsion größer als 1 und kleiner als 1,6 ist.

## Claims

1. Use of a perfluorocarbon (PFC)-mixture containing at least one liquid perfluorocarbon except perfluorophenanthrene, which is converted by a purification process into a compound which is free from C=C-double bonds and C-H-bonds, for producing a treatment liquid for reapplying (unfolding) detached retaina to the chorioid of the eye.

2. Use according to claim 1 characterised in that the PFC-mixture contains at least one PFC from the group perfluorodecaline, perfluorocyclohexylmorpholine, perfluorooctane and perfluorooctylbromide.

3. Use according to claim 2 characterised in that the PFC from the group perfluorodecaline, perfluorocyclohexylmorpholine, perfluorooctane and perfluorooctylbromide is present in a proportion of 96 to 99. 9% by volume.

4. Use according to one of claims 1 to 3 characterised in that the PFC-mixture has a proportion of 1 to 4% by volume of a perfluorodimorpholinoalkane or a perfluorinated tricyclic compound.

5. Use according to claim 4 characterised in that the perfluorinated tricyclic compound is perfluoroperhydrophenanthrene.

6. Use according to claim 4 characterised in that the alkane is perfluorodimorpholinobutane or perfluorodimorpholinopropane.

7. Use according to one of claims 1 to 6 characterised in that a gas is dissolved in the PFC-mixture.

8. Use according to claim 7 characterised in that the dissolved gas serves for tissue care.

9. Use according to claim 8 characterised in that the gas dissolved in the PFC-mixture is oxygen.

10. Use according to claim 7 characterised in that the dissolved gas serves to adjust the contact pressure involved in the retina unfolding operation.

11. Use according to claim 10 characterised in that one or more gaseous PFCs or SF₆ are dissolved in the PFC-mixture.

12. Use according to one of claims 1 to 11 characterised in that it is in the form of a PFC-emulsion in water.

13. Use according to claim 12 characterised in that the emulsion is adjusted in respect of its osmotic and oncotic pressure to eye vitreous humour conditions.

14. Use according to claim 12 or claim 13 characterised in that the emulsion is adjusted in respect of its osmotic and oncotic pressure to chamber fluid conditions.

15. Use according to one of claims 10 to 14 characterised in that the amount of PFC-mixture in the emulsion is 10 to 60% by weight.

16. Use according to one of claims 10 to 15 characterised in that the emulsifier is a non-ionogenic emulsifier.

17. Use according to one of claims 10 to 16 characterised by a free emulsifier concentration of 1 to 3%.

18. Use according to one of claims 10 to 17 characterised in that the emulsifier is a non-iogenic biocompatible tenside.

19. Use according to claim 18 characterised in that the tenside is an ethylene oxide-propylene oxide block polymer or a derivative derived therefrom.

20. Use according to one of claims 10 to 19 characterised in that the osmotic pressure is adjusted by physiological salt components.

21. Use according to one of claims 10 to 20 characterised in that the oncotic pressure is adjusted by the addition of at least one substance from the group hydroxyethyl starch, dextran and hyaluronic acid.

22. Use according to one of claims 10 to 21 characterised in that the specific weight of the emulsion is greater than 1 and lower than 1.6.

## Revendications

1. Utilisation d'une mixture de perfluorocarbones (PFC), comportant au moins un perfluorocarbone liquide, perfluorophénanthrène excepté, transformée dans un processus d'épuration en un composé exempt de doubles liaisons C=C et de liaisons C-H, pour la préparation d'un liquide thérapeutique destiné à la réapplication (déplissage) d'une rétine décollée sur la choroïde de l'oeil.

2. Utilisation suivant la revendication 1, caractérisée en ce que la mixture de PFC présente au moins un PFC du groupe perfluorodécaline, perfluorocyclohexylmorpholine, perfluorooctane, perfluorooctylbromure.

3. Utilisation suivant la revendication 2, caractérisée en ce que le PFC du groupe perfluorodécaline, perfluorocyclohexylmorpholine, perfluorooctane, perfluorooctylbromure, est contenu en une proportion de 96 à 99,9 % en volume.

4. Utilisation suivant l'une des revendications 1 à 3, caractérisée en ce que la mixture de PFC présente une proportion de 1 à 4 % en volume d'un perfluorodimorpholinoalcane ou d'un composé tricyclique perfluoré.

5. Utilisation suivant la revendication 4, caractérisée en ce que le composé tricyclique perfluoré est du perfluoroperhydrophénanthrène.

6. Utilisation suivant la revendication 4, caractérisée en ce que l'alcane est du perfluorodimorpholinobutane ou du perfluorodimorpholinopropane.

7. Utilisation suivant l'une des revendications 1 à 6, caractérisée en ce qu'un gaz est dissous dans la mixture de PFC.

8. Utilisation suivant la revendication 7, caractérisée en ce que le gaz dissous sert à la nutrition tissulaire.

9. Utilisation suivant la revendication 8, caractérisée en ce que de l'oxygène est dissous en tant que gaz dans la mixture de PFC.

10. Utilisation suivant la revendication 7, caractérisée en ce que le gaz dissous sert au réglage de la pression lors du déplissage de la rétine.

11. Utilisation suivant la revendication 10, caractérisée en ce qu'un ou plusieurs PFC gazeux ou du SF₆ sont dissous dans la mixture de PFC.

12. Utilisation suivant l'une des revendications 1 à 11, caractérisée en tant qu'émulsion de PFC dans l'eau.

13. Utilisation suivant la revendication 12, caractérisée en ce que l'émulsion est réglée sur les conditions du corps vitré, quant à sa pression osmotique et oncotique.

14. Utilisation suivant l'une des revendications 12 et 13, caractérisée en ce que l'émulsion est réglée sur les conditions de l'humeur aqueuse, quant à sa pression osmotique et oncotique.

15. Utilisation suivant l'une des revendications 10 à 14, caractérisée en ce que la part de mixture de PFC dans l'émulsion est de 10 à 60 % en poids.

16. Utilisation suivant l'une des revendications 10 à 15, caractérisée en ce que l'émulsifiant est un émulsifiant non-ionique.

17. Utilisation suivant l'une des revendications 10 à 16, caractérisée par une concentration libre d'émulsifiant de 1 à 3 %.

18. Utilisation suivant l'une des revendications 10 à 17, caractérisée en ce que l'émulsifiant est un agent tensioactif biocompatible, non ionogène.

19. Utilisation suivant la revendication 18, caractérisée en ce que l'agent tensioactif est un polymère bloc oxyde d'éthylène-oxyde de propylène, ou un dérivé de ce dernier.

20. Utilisation suivant l'une des revendications 10 à 19, caractérisée en ce que le réglage de la pression osmotique est assuré par des composants salins physiologiques.

21. Utilisation suivant l'une des revendications 10 à 20, caractérisée en ce que la pression oncotique est réglée par addition d'une substance au moins du groupe hydroxyéthylamidon, dextrane, acide hyaluronique.

22. Utilisation suivant l'une des revendications 10 à 21, caractérisée en ce que le poids spécifique de l'émulsion est supérieur à 1 et inférieur à 1,6.
